# EUROPEAN PATENT SPECIFICATION

(11) **EP 0 622 049 B1**
(45) Date of publication and mention of the grant of the patent: **09.12.1998**
(21) Application number: 94303139.3
(22) Date of filing: 29.04.1994
(51) Int. Cl.: A61B 17/12, A61B 17/068, A61B 17/072

(54) **Mechanism for use by endoscopic and open procedures**
Mechanismus zur Verwendung bei endoskopischen und offenen Eingriffen
Mécanisme pour adaptation à des procédures endoscopiques ou ouvertes

(30) Priority: 29.04.1993 US 54732
(43) Date of publication of application: 02.11.1994
(73) Proprietor: ETHICON, INC., Somerville, N J 08876 (US)
(72) Inventor: Paul, Michel A., Cincinnati, Ohio 45230 (US); Chuisano, Michael P., Cincinnati, Ohio 45249 (US); Goolie, Gary R., West Chester, Ohio 45069 (US); Wise, Kim A., Loveland, Ohio 45140 (US)
(74) Representative: Fisher, Adrian John

(56) References cited:
- EP-A- 0 433 581
- EP-A- 0 484 677
- GB-A- 2 103 936
- US-A- 5 174 487

## Description

### FIELD OF THE INVENTION

This invention relates to surgical mechanisms. More particularly this invention relates to surgical mechanisms capable of being converted from being useful for open endoscopic procedures to being useful for procedures at a moment's notice.

### BACKGROUND OF THE INVENTION

Recently, there have been quite a few important technical advances in the surgical field. Through the 1980's, all of these advances were in the field of open surgical procedures. That is, the advances in the surgical field were accomplished in procedures which involved the cutting open of the body through large incisions, and the use of medical instruments within this large incision, with the body open. Thus, these procedures placed very little emphasis on the size of the instruments used, because the incision was large and such instruments could be used in this large volume. The head size of the instruments (in these cases) were never larger than the opening.

In more recent years, there has been a revolution in the surgical field to procedures which are accomplished endoscopically or laparoscopically. These procedures can be accomplished so that a very small incision is made. Usually, an incision is made by a trocar so that the trocar punctures a portion of the body. For instance, in laparoscopic procedures the abdomen is punctured. Thereafter, surgical instruments are inserted through the trocar cannula so that the procedure can take place through the trocar and within the "closed" volume of the body.

Necessarily, these latter procedures rely on devices with reduced size. Because the procedure takes place through a reduced size incision and a reduced size cannula, an instrument head must be capable of fitting through the incision and cannula. In this fashion, an emphasis has been placed on the head of the device, with size itself placed at a premium.

Until now, endoscopic instruments would generally not be used in open procedures and, certainly, open procedure instruments could not be used laparoscopically. Thus, two separate procedural fields have been created. In fact, much emphasis has been placed on endoscopic procedures; in some instances, there have been more advances in endoscopic procedures as compared to the formerly very common open procedures.

Nonetheless, there remains a recognized need in the medical community. This need exists in instances where the surgeon inadvertently causes bleeding during an endoscopic procedure. When there is this inadvertent bleeding, it is frequently necessary to convert the procedure from an endoscopic procedure to an open procedure immediately. This involves, of course, the creation of a large incision within the body, and the use of instruments to control the bleeding which has been caused endoscopically.

Frequently, when there is such an event which occurs, the time of conversion from endoscopic to an open procedure can be hectic, even hysterical. The hysteria results from a need to get to the bleeding part of the patient as quickly as possible. This is true for a number of reasons. First, any unnecessary or inadvertent bleeding is undesirable. Second, because the bleeding may be coming from an unknown area, the patient's well-being could seriously be at stake. Third, because the surgeon is now attempting to control the bleeding during the opening up of the patient's body, there is the potential for trauma and/or time delay, which may result in additional trauma to the patient. As none of these possibilities or inadvertencies are desirable, it becomes extremely necessary to quickly, reliably and repeatably be able to convert the procedure from an endoscopic procedure to an open procedure.

Surgical interns are indeed taught how to convert an endoscopic procedure to an open procedure. In the operating room, provisions are made to convert the procedure from an endoscopic to an open procedure. In the operating room itself, during an endoscopic procedure, various trays of equipment are arrayed before the surgeon. These trays enable the surgeon to perform the endoscopic procedure. These trays include, among other things, trocars, instruments such as graspers, dissectors and forceps useful in endoscopic proceedings, clip appliers, staplers, needle holder, scalpels, etc. All these instruments are capable of being placed through a trocar cannula. Their use is observable on a video screen such as those typically and commonly used during endoscopic procedures.

Also contained within the operating room are instruments useful for an open surgical procedure. These may be for instance, larger head staplers, clip appliers, graspers and forceps. There also may be retractors, scalpels, electrocautery units, open heart mechanisms or the like. Each of these groups of materials are kept on separate stands within the operating room. Then, if there is a need to convert a procedure from an endoscopic procedure to an open procedure, the endoscopic mechanisms are wheeled away from the surgeon and the open surgical procedure mechanisms are placed in front of the surgeon. Thus, a rather time consuming and expensive endeavor occurs wherein the devices are wheeled away and new devices are used. Also, the necessity arises to have multiple devices themselves within the operating room. Often, this results in a pure space filling problem.

The Applicant has realized that some of the exigencies of this situation can be avoided. This can be done with the following in mind. A commonality of instruments might be useful to be provided to the surgeon. For instance, if a mechanism were available wherein the surgeon could go from an endoscopic procedure to an open procedure with impunity, it would provide for better coverage for both the surgeon and the patient. Also, if the surgeon has the capability of using various length instruments within the endoscopic procedure, or of using various instruments within the endoscopic procedure while using the same essential apparatus, this might prove useful. Further, during an open procedure if there could be some commonality of instruments and interests, this may prove quite functionally desirable for the surgeon's needs and capabilities.

Handles with disposable shafts are known from the prior art, EP-A-0484677 for example discloses an endoscopic stapler in which the endoscopic portion of the instrument is detachable from the frame and handle portion of the instrument, said handle portion being reusable with replacement endoscopic portions.

### SUMMARY OF THE INVENTION

The needs expressed above are alleviated in part due to a new and useful system created with the surgeon in mind, especially during the exigent situation of conversion to an open procedure from an endoscopic procedure. What is described herein is a system wherein a common handle can be attached to either an endoscopic instrument or an open surgical instrument. For instance, a common handle may have attached to it an open surgical clip applier. Or, this may have attached to it an endoscopic clip applier. Or, there may be attached to it an endoscopic stapler. Or, there may be attached an open stapler. This capability continues across all procedures and product lines. What is necessary is that the surgeon has on hand, at virtually an instance, the capability of converting the procedure from an endoscopic one to an open one, or converting one instrument to another without having to change an essential part, the handle used in the device. With the common handle, also, there can be reuse of the handle, with disposal of the cartridge held therein. Of course, the cartridge can be either an open procedure surgical cartridge or an endoscopic cartridge.

The cartridge held within the handle can be compatible with a trocar or can be useful outside a trocar. Of course, such a cartridge, if useful for an endoscopic use, could be used by the surgeon for open use, but with the availability of an open procedure cartridge nearby, there is no need for such an application. Of course, because the handle is common to all uses, a surgeon can have on a surgical tray cartridges for all of the instruments necessary to have either an open procedure or an endoscopic procedure. Thus, some of the hectic disarray involved in converting a surgical procedure is done away. That is, there is no need to wheel away the instruments useful for an endoscopic procedure, and wheel in front of the surgeon the instruments useful for an open procedure. Now, the surgeon has all the instruments on one tray, as embodied in the cartridges useful with the common handle. With such an invention in place, the length of the procedure can be shortened, and the time to convert procedures is dramatically altered. Also, the surgeon will feel more confident is using a single tray to accomplish a procedure, rather than having to rely on placement of a set of devices within the operating room, but not directly in front of the surgeon.

This invention will be better understood in association with the attached Description of the Drawings and the Detailed Description of the Invention.

### DESCRIPTION OF THE DRAWINGS

Figure 1 shows an endoscopic surgical device useful with this mechanism;
Figure 2 shows a handle of this mechanism;
Figure 3 shows an open procedure cartridge useful with the handle of this mechanism described in Figure 2;
Figure 4 is an alternate embodiment of this invention;
Figure 5 is another additional alternate embodiment of this invention;
Figure 6 is a third embodiment of this invention; and
Figure 7 is a fourth alternate embodiment of this invention.

### DETAILED DESCRIPTION OF THE INVENTION

As seen in this invention there is described an endoscopic clip applier 19. This clip applier contains a series of clips as is better described in U.S. Patent No. 5,171,249. These clips are stored in a shaft 15 and the shaft is placed on a handle 20. This handle 20 is further described in the same patent.

Uniquely, the shaft 15 of the present device is detachable from the handle 20 of this device 19. Thus, the firing mechanism 25 and the clip feeding mechanism 30 as contained in the shaft portion of the device are detachable from the handle portion 20 of this device by conventional attachment means.

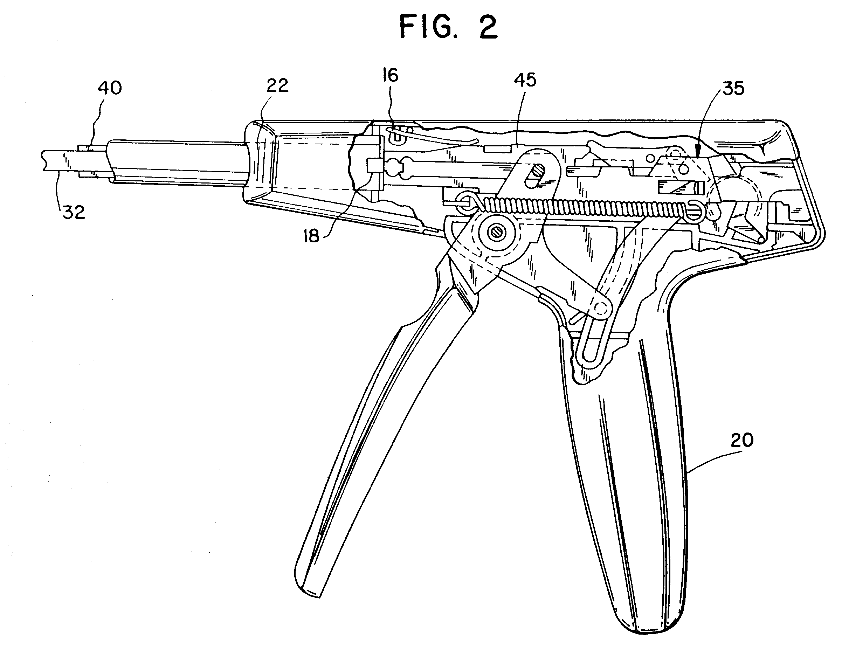

This attachment is better seen in Figure 2. As seen in Figure 2, cam channel 32 fits into firing shaft 35. Also, feed channel 40 (for loading clips) fits into feed shaft 45. Feed shaft 45 and firing shaft 35 are operable to cover reciprocation of feed channel 40 and cam channel 30 respectively. Collar 16 fits into handle 20, to hold shaft 15 in handle 20 during operation. To remove shaft 15, collar 20 is rotated so that key 18 fits through notch 22.

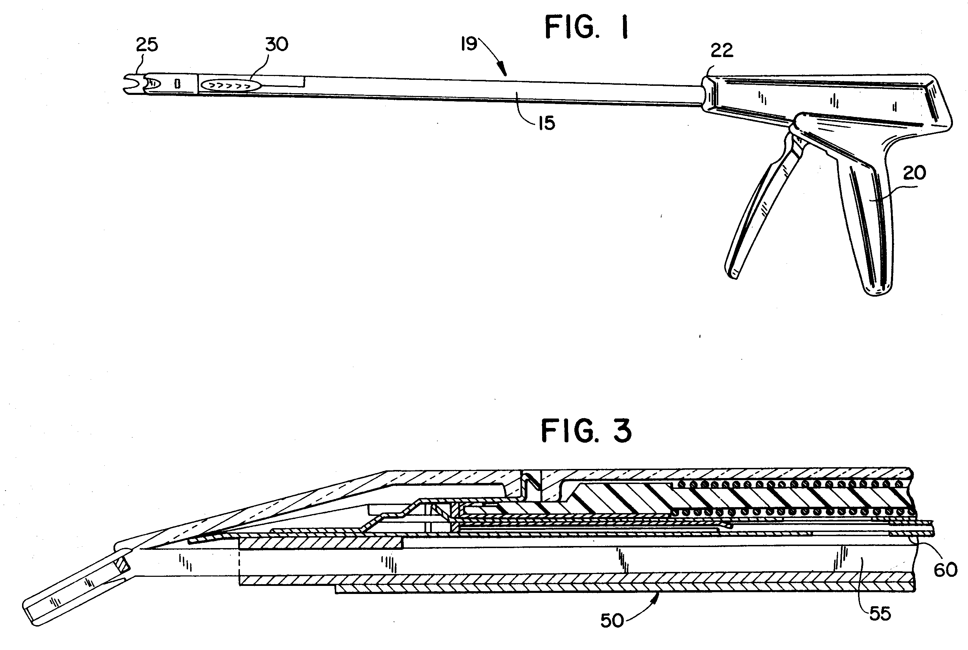

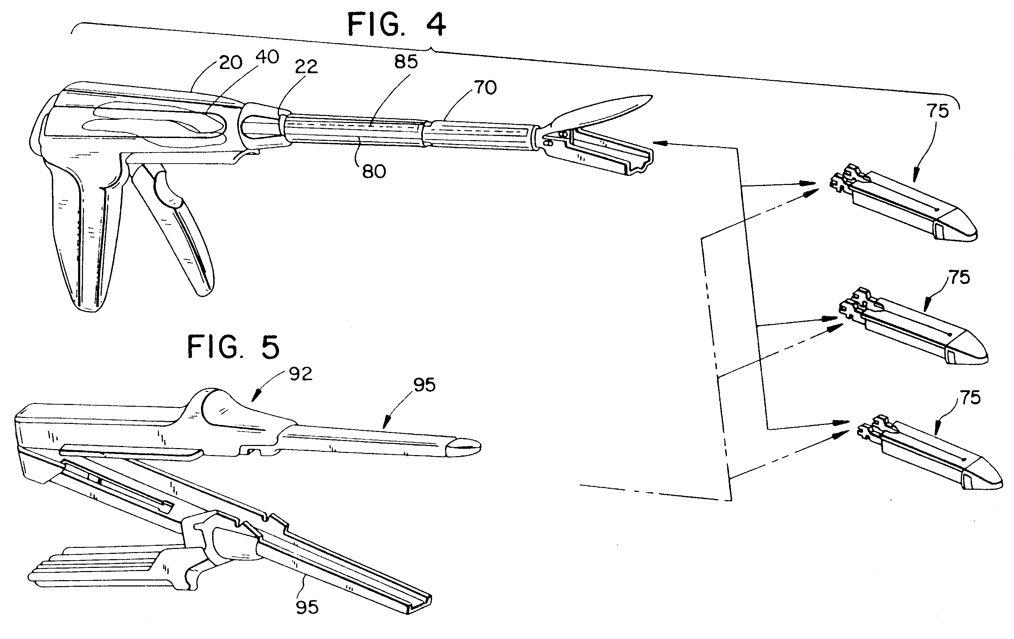

Figure 3 describes an open procedure ligating shaft 50. This shaft corresponds in many ways to a ligating clip applier shaft disclosed in European Patent Appln. EP-A-630616 entitled "Ligating Clip Applier" (END-37), filed this day and claiming priority from USSN 54734 except that this mechanism is in cross circular section, to mate with handle 20. This ligating shaft 50 is capable of being inserted into the handle 20 of the instrument 19 in Figure 1. Then, the clip applying 55 and feeding members 60 of shaft 50 are capable of mating with the clip applying firing shaft 35 and feeding shaft member 45, respectively, of the device in Figure 2, so that this device is entirely useful as an open surgical device. Mating is accomplished by known connecting means such as, for instance, by bayonet mounting the pieces one into the other. Locking is accomplished by rotating shaft 50 in handle 20, as described in connection with Figures 1 and 2.

What is understood is that now, whenever the surgeon desires to have an instrument at the ready, he can continue to use the handle 20 of this device. This handle 20 is capable of having interchanged within it staplers and ligating clip appliers. Further, this handle can have inserted into it open or endoscopic devices. Now, the surgeon can have one series of instruments useful to be interchanged with the handle 20. Then, if the surgery must go from an endoscopic surgery to an open procedure, the surgeon merely removes the endoscopic device (as in Figure 1) from a trocar, removes the shaft 15 from its handle 20, and inserts open clip applier shaft 50, for instance.

Figure 4 describes an endoscopic stapling shaft 70 of this invention. The endoscopic shaft 70 has on its end a stapler cartridge 75, similar to the cartridge described in U.S. Patent US-A-5307976 (Serial No. 917,636). This device is capable of being placed into the handle portion 20 of Figure 1, so that the handle portion 20 containing feed shaft 45 and firing shaft 35 mates (seen in phantom in Figure 4) with the staple applying 80 mechanisms and clamping mechanisms 85, as seen in Figure 4. Again, mating is accomplished by known connection mechanisms, like a socket and ball mechanism. Figure 5 shows an open staple applying mechanism 90 comparable to that of the instrument of Figure 4, and further comparable to the instrument of U.S. Patent No. 4,633,861. This device is also capable of mating with the handle 20 of Figure 1. Furthermore, the cartridge 75 in Figure 4 is capable of being emplaced into the jaws 95 of the stapler of Figure 5. Thus, where heretofore there have been separate cartridges useful for either open or endoscopic procedures, now there are cartridges capable of being used in either procedure.

What is understood is that now, whenever the surgeon desires to have an instrument at the ready, he can continue to use the handle 20 of this device. This handle is capable of having interchanged within it staplers and ligating clip appliers. Further, this handle can have inserted into it open or endoscopic devices. Now, the surgeon can have one series of instruments useful to be interchanged with the handle 20. Then, if the surgery must go from an endoscopic surgery to an open procedure, the surgeon merely removes the endoscopic device (as in Figure 1) from a trocar, removes the shaft 15 from its handle 20, and inserts the endoscopic shaft 70 or staple applying mechanism 90. What is entirely important is now the surgeon has an immediate usage the capability of going from an endoscopic to an open procedure without even thinking. This advancement will allow the surgeon to be able to more expeditiously convert the procedure, and even more expeditiously finish the surgical procedure. Now, the capability is given to the surgeon so that the surgeon may remove a ligating endoscopic device 10 and inserting an endoscopic stapling device 70. Or, the surgeon may keep the ligating device 10 inserted through one trocar tube, and detach the handle 20 so that the handle 20 can be attached to a stapler 70 contained in another trocar. Or, the surgeon may wish to dispose of all the used shafts, and have the handle 20 autoclaved, while using a new set of shafts for another patient.

For instance, it is intended to include cartridges which incorporate staples, absorbable fasteners, fibrin glue, adhesive, clips and the like, well known in the art.

Importantly, the cartridge of these devices should be useful with either an open stapling procedure or a closed endoscopic procedure. Previously, such cartridges have been created so that they are useful one for specific types of procedure. Now, these cartridges extend across procedural lines, so that their versatility is enhanced. For instance, it is intended to include cartridges which incorporate staples, absorbable fasteners, fibrin glue, adhesive, clips or the like, all of which are well known in the art.

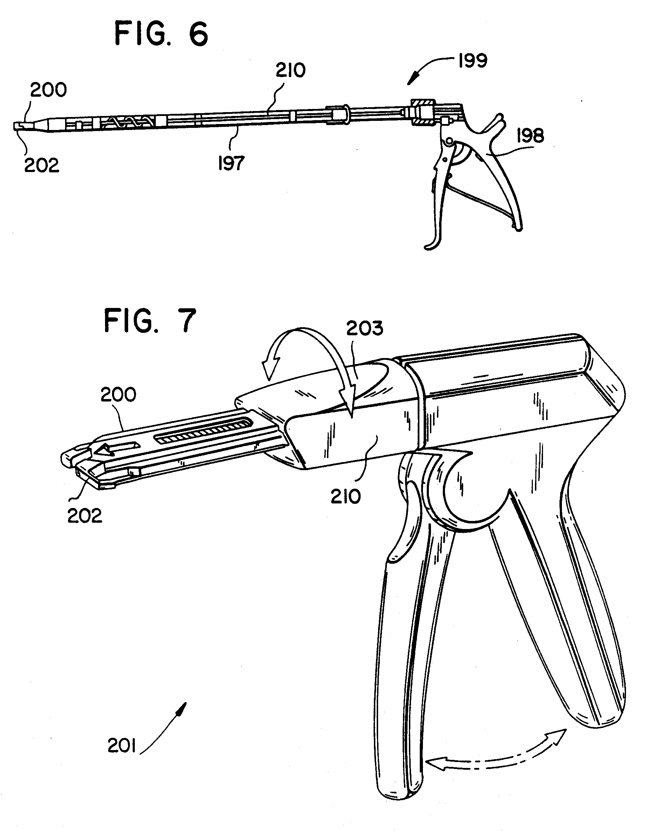

Figure 6 describes an endoscopic hernia stapler 199 similar in many ways to the instrument of U.S. Patent No. 5,174,487. Figure 7 describes a skin stapler 201 similar to the skin stapler described in U.S. Patent No. 5,161,725. Again, the cartridge 200 of the two instruments is common. Now cartridge 200 containing staples 202 may be inserted into either handle 198 on shaft 197 or handle 203 of the instruments 199, 201 respectively. Thus, like the previous instruments, the interchangeable cartridge 200 allows the cartridge 200 to be used with either an endoscopic or an open application. Mating is similar to the previous embodiments, in that push bar 210 fits into cartridge 200. Push bar 210 is an element of both staplers 199, 201, and mates with cartridge 200 in each instance, by known attaching mechanisms. It is this departure from the art which provides the new and useful application of this mechanism.

What is entirely necessary is that the surgeon now has the capability of going back and forth from open to endoscopic procedures, from ligating clip appliers to staplers, and vice versa. This increased capability is quite substantial, and is considered the subject matter of the claims attached hereto. It is to be understood that this invention may contain many equivalents, and their scope is to be intended to be covered by the scope of these claims.

## Claims

1. A surgical handle (20) in combination with a plurality of surgical shafts (15, 50), each of said shafts (15, 50) being mateable with said surgical handle (20), and at least a first one of said shafts (15, 50) being useful for accomplishing a step in an endoscopic procedure, characterised in that a second one of said shafts (15, 50) provides a functionality which is different from the functionality provided by said first shaft, said second shaft being useful for accomplishing one of a different step in said procedure or a step in an open surgical procedure.

2. The combination of claim 1 wherein one of said shafts enables a stapling step.

3. The combination of claim 1 wherein one of said shafts enables a ligating step.

4. The combination of claim 1 wherein one of said shafts enables a grasping step.

5. The combination of claim 1 wherein one of said shafts enables a dissecting step.

6. The combination of claim 1 wherein one of said shafts enables a cutting step.

7. The combination of claim 1 wherein one of said shafts is circular.

8. The combination of claim 1 wherein one of said shafts enables an adhesion step.

## Patentansprüche

1. Chirurgischer Handgriff (20) in Kombination mit einer Vielzahl chirurgischer Einsatzstücke (15, 50), wobei jedes der Einsatzstücke (15, 50) in den chirurgischen Handgriff (20) einsetzbar und mindestens eines der Einsatzstücke (15, 50) zur Duchführung eines Schrittes bei einem endoskopischen Eingriff verwendbar ist, dadurch gekennzeichnet, daß ein zweites der Einsatzstücke (15, 50) mit einer anderen Funktion als das erste Einsatzstück zur Durchführung eines anderen Schrittes bei diesem Eingriff oder eines Schrittes bei einem offenen chirurgischen Eingriff verwendbar ist.

2. Kombination von Anspruch 1, bei welcher eines der Einsatzstücke einen Klammerschritt ermöglicht.

3. Kombination von Anspruch 1, bei welcher eines der Einsatzstücke einen Abschnürungsschritt ermöglicht.

4. Kombination von Anspruch 1, bei welcher eines der Einsatzstücke einen Greifschritt ermöglicht.

5. Kombination von Anspruch 1, bei welcher eines der Einsatzstücke einen Trennschnitt ermöglicht.

6. Kombination von Anspruch 1, bei welcher eines der Einsatzstücke einen Schnitt ermöglicht.

7. Kombination von Anspruch 1, bei welcher eines der Einsatzstücke rund ist.

8. Kombination von Anspruch 1, bei welcher eines der Einsatzstücke einen Klebeschritt ermöglicht.

## Revendications

1. Une poignée chirurgicale (20) en combinaison avec une pluralité de corps d'instrument chirurgical (15, 50), chacun desdits corps (15, 50) pouvant être adapté à ladite poignée chirurgicale (20) et un premier au moins desdits corps (15, 50) étant utilisable pour effectuer une étape au cours d'une intervention endoscopique, caractérisée en ce qu'un second desdits corps (15, 50) remplit une fonction qui est différente de la fonction remplie par ledit premier corps d'instrument, ledit second corps servant à accomplir une étape différente de ladite intervention ou une étape au cours d'une intervention chirurgicale ouverte.

2. La combinaison de la revendication 1 dans laquelle l'un desdits corps d'instrument permet l'exécution d'une étape d'agrafage.

3. La combinaison de la revendication 1 dans laquelle l'un desdits corps d'instrument permet l'exécution d'une étape de formation de ligature.

4. La combinaison de la revendication 1 dans laquelle l'un desdits corps d'instrument permet l'exécution d'une étape de préhension.

5. La combinaison de la revendication 1 dans laquelle l'un desdits corps d'instrument permet l'exécution d'une étape de dissection.

6. La combinaison de la revendication 1 dans laquelle l'un desdits corps d'instrument permet l'exécution d'une étape d'incision.

7. La combinaison de la revendication 1 dans laquelle l'un desdits corps d'instrument est circulaire.

8. La combinaison de la revendication 1 dans laquelle l'un desdits corps d'instrument permet l'exécution d'une étape d'adhésion.
